# EUROPEAN PATENT APPLICATION

(11) **EP 1 970 008 A1**
(43) Date of publication of application: **17.09.2008**
(21) Application number: 06834445.6
(22) Date of filing: 11.12.2006
(51) Int. Cl.: A61B 5/151

(54) **PUNCTURE NEEDLE DEVICE**

(30) Priority: 12.12.2005 JP 2005357945
(71) Applicant: Nichinan Corporation, Ayase-shi, Kanagawa 252-1125 (JP)
(72) Inventor: HORIE, Katsuto, Ayase-shi Kanagawa 252-1125 (JP)
(74) Representative: Müller, Frithjof E.
(86) International application number: PCT/JP2006/324690
(87) International publication number: WO 2007/069572

(57) **Abstract**

Disclosed is a pricking needle device, termed a lancet, used by a patient with diabetes in sampling his/her own blood to measure his/her blood sugar value. The pricking needle device includes a pricking member body (12) and a needle end protective cap (13). The pricking member body, formed of synthetic resin, carries a pricking needle (11), the distal end of which is protruded from the end face of the pricking member body. The needle end protective cap, equally formed of synthetic resin, is coupled to the pricking member body, as a protruded distal end of the needle on the pricking member body (12) is admitted into the inside of the needle end protective cap. The pricking needle device includes a position registration unit (34), (35), a coupling unit, made up of engagement segments (21), (22) and engagement grooves (23), (24), and a needle end housing section (38). The position registration unit guides the pricking member body to a preset location of the needle end protective cap for position registration when the pricking member body (12) and the needle end protective cap (13) are to be coupled together. The pricking member body and the needle end protective cap, position registration of which has been made by the position registration unit, are then rotated relative to each other for engaging the engagement segments with the engagement grooves. The needle end housing section is provided on the needle end protective cap and accommodates the needle, protruded from the end face of the pricking member body, in a hermetically sealed state.

## Description

### Technical Field

This invention relates to a pricking needle device, known as a lancet, used by a patient with diabetes to measure his/her own blood sugar value. More particularly, this invention relates to a pricking needle device in which the needle end used by the patient with diabetes for pricking his/her finger end and having the blood still affixed thereto is covered by a needle end protective cap to prevent the blood from being scattered to outside or from becoming affixed to an external object, whereby the needle and the cap may be discarded in safety.

### Background Art

Recently, the number of patients with diabetes, who sample minor amounts of the blood for themselves, measure their blood sugar values using blood sugar value meters for household use, and who control their blood sugar values to a desirable level as they use diet and exercise therapies in combination with insulin injection, if necessary, is increasing veritably from year to year.
The background for this may be such that the number of patients with diabetes is increasing recently and, in addition, that the validity of blood sampling as well as management and control of the blood sugar values by the patients themselves has been confirmed and recommended by medical practitioners. In addition, such a situation needs to be taken into account that, with improvement in reliability and operability of the blood sugar value meter, precision in measurement has been improved, while the volume of the blood needed for measurement has decreased significantly. With decrease in the volume of blood needed for measurement, the needle used for blood sampling is becoming thinner in diameter. The needle used in early pricking needle devices is on the order of 0.8 mm in diameter, whereas, with most of needles, used in these days, the diameter is on the order of 0.4 mm. With decreasing diameter of the needle, used for pricking, the pain felt in pricking the finger end, or the size of the wound, produced by pricking, is decreasing, and hence the burden on the mental and physical states of the patient has also decreased significantly.
The graveness of the effect of rampancy of AIDS or hepatitis, mediated by the blood, on the society at large, and the severe disturbance in the daily lives of the patients with these diseases, have come to be recognized in general. This also accounts for the severe demand raised from the perspective of environment protection in connection with the handling of medical implements to which blood still remains affixed.
To cope with this social demand, such a pricking needle device, in which the needle end is automatically accommodated in a needle end protective cap, a needle projecting mechanism is rendered immobile and disabled to be used, and in which the device may directly be discarded in safety, is now in use in medical organizations, such as hospitals. An example of this sort of the needle pricking device has been described in USP 5,755,733 (Patent Publication 1).
As a pricking needle device for domestic use, such a device, disposable practically in safety, in which the needle end, having the blood affixed thereto after use, is reliably covered with a needle end protector, has been provided, and has come to be used in increasing quantities. An example of this sort of the needle pricking device is described in USP 5,385,571 (Patent Publication 2).
The pricking needle device, disclosed in Patent Publication 2, includes a pricking member body, formed of synthetic resin, and a needle end protective cap, equally formed of synthetic resin. The pricking member body includes a needle adapted to be used for pricking the finger end. The distal end of the pricking member body, carrying the needle, is accommodated in the needle end protective cap. This needle end protective cap includes a housing section in which to accommodate the distal end of the pricking member body. A ring-shaped engagement projection is formed on the inner rim of the opening side of the housing section. The outer rim of the distal end of the pricking member body is formed with four retention protrusions.
If, in the above-described pricking needle device, the pricking member body, carrying the needle, to the end of which is still affixed the blood, after pricking the finger end, and the needle end protective cap, are to be coupled together, the needle end protective cap is set on a setting site, such as a table, with the opening side of the housing section directing upwards. The pricking member body, carrying the needle, to the end of which is still affixed the blood, after pricking the finger end, is gripped with the finger end, and the distal end of the pricking member body, from which is protruded the needle end, is admitted into the housing section of the needle end protective cap. As the distal end side of the pricking member body is progressively admitted from the opening side into the inside of the needle end protective cap, the retention projections on the outer rim of the distal end side of the pricking member body ride over the engagement projections on the inner rim of the opening so as to be intruded into the housing section. As the pricking member body is intruded further, the retention projections are admitted into and engaged with the ring-shaped engagement groove which is formed within the housing section of the ring-shaped retention projections
The pricking member body and the needle end protective cap are unified together by the retention projections on the pricking member body engaging in the engagement groove in the needle end protective cap. With the pricking member body and the needle end protective cap thus unified together, the needle provided on the pricking member body is accommodated in the housing section of the needle end protective cap, with the result that the blood affixed to the needle end may be prevented from being scattered to outside the device or from becoming affixed to human beings or outside objects.
Meanwhile, the coupling of the above-described coupling system is by snap fit between the pricking member body and the needle end protective cap. This snap-fit coupling system takes advantage of properties of the crystalline resin. That is, the coupling is by taking advantage of the properties of the crystalline resin, the pricking member body and the needle end protective cap are formed of, that is, the properties that the resin is slightly elastically deformed when the two members formed of the resin are fitted to each other.

### Disclosure of the Invention

### Problem to be solved by the invention

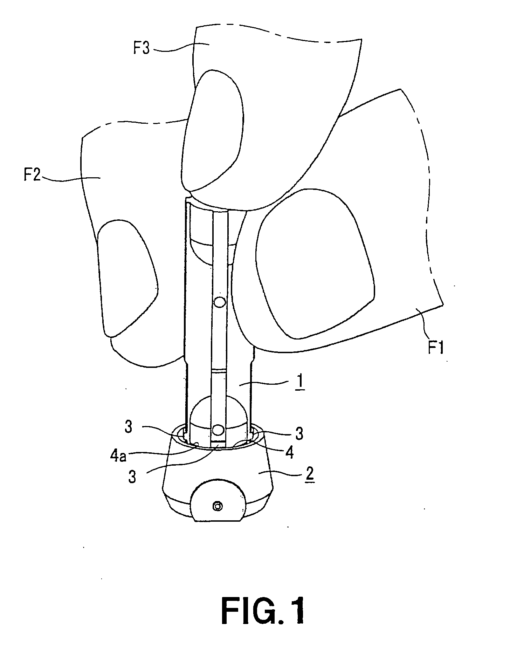

If, in the pricking needle device, scattering of the blood affixed to the needle end to outside is to be prevented positively, it is necessary to reliably connect the pricking member body and the needle end protective cap to each other to unify the two members against possible detachment.
If, in the pricking needle device of the snap fit coupling system, the pricking member body and the needle end protective cap are to be reliably coupled together, a high coupling force needs to be used. If, in the snap-fit coupling system, the coupling force is increased, the force needed for admission is increased, thus deteriorating the operability in the fitting coupling between the pricking member body and the needle end protective cap.
In actuating the pricking needle device, disclosed in Patent Publication 2, a pricking member body 1 is gripped, to admit the pricking member body 1 into the needle end protective cap 2 and to connect them together, as shown in Fig.1. That is, the pricking member body 1 is held as its lateral side is retained by a thumb finger F1 and as the mid finger or the fourth finger F2 is also used for holding, as shown in Fig.1. It is necessary that the index finger F3 or the mid finger is applied to the rear end of the pricking member body 1 and mainly the index finger applied to the rear end of the pricking member body 1 is used to generate the force applying the pricking member body 1 onto a needle end protective cap 2 for inserting the pricking member body into the needle end protective cap. By so doing, the pricking member body 1 may be admitted into the needle end protective cap 2 in such a manner that a retention projection 3 on the pricking member body 1 will ride over the engagement projection on the inner rim of an opening part 4a of a housing section 4 of the needle end protective cap 2.
If the needle has pricked one of the three fingers, gripping the pricking member body 1, for blood sampling, the wound is as yet not healed and the pain persists. If the pricking member body 1 is inserted in this state into the needle end protective cap 2, considerable pain will be inflicted on the user. In particular, in a pricking needle device in which significant force is needed interconnect the pricking member body 1 and the needle end protective cap 2 against inadvertent detachment, a significant force of insertion is required, thus causing severe pain to the user. To avoid such pain, the user would be compelled to use the other hand the fingers of which remain intact. If this other hand is not the user's dominant hand, the user would feel it difficult to interconnect the two members.
To relieve the physiological pain the blood sampling from the own finger end would inflict on the user, and his/her inconvenience in everyday life, a method for measuring the blood sugar value from his/her perspiration or saliva has so far been researched and proposed. However, this method is far inferior to the method of blood sampling from the capillary vessel of the finger end. Under this situation, blood sampling from the finger end by the user necessarily becomes the mainstream in self-monitoring of blood glucose (SMBG) in households.
To relieve the pain caused by pricking, it has also been proposed to sample the blood not from the finger end but from the forearm. It has however been medically confirmed that the blood sugar value of the blood sampled from the forearm is not necessarily coincident with that of the vein serum, depending on the sampling time zone. Hence, the blood sampling from the capillary vessel of the finger end is used as customary blood sampling method.
That is, in measuring the blood sugar value, the blood sampled from the finger end is most routinely used. Under this condition, relieving the load imposed on the patient's finger end is strongly desired, in connection with assuring the safety of the needle end of the pricking needle device after blood sampling, and in connection with improving and maintaining the quality of life (QOL) of a user in need of four to seven blood sampling operations per day, as in the case of a patient with Type I diabetes (insulin-dependent diabetes mellitus (IDDM)).
In addition, it is strongly desired to relieve the pain or burden not only of I-diabetics but also of a person who is in need of blood sampling from his/her finger end.
It is therefore an object of the present invention to provide a pricking needle device with which a needle used for pricking the finger end of a person in need of blood sampling from the finger end and having the blood still affixed thereto may readily be housed in a needle end protective cap without inflicting pain on the person who uses the device for blood sampling.
It is another object of the present invention to provide a pricking needle device with which a needle used for pricking and having the blood still affixed thereto may readily be housed reliably in safety in the needle end protective cap and with which the blood affixed to the needle may reliably be prevented from leaking to outside.
A pricking needle device according to the present invention comprises a pricking member body of synthetic resin, carrying a pricking needle, with the distal end of said pricking needle projected from an end face of the pricking member body, and a needle end protective cap of synthetic resin, into which the foremost part of the pricking member body, the distal end of the needle is protruded from, is inserted for coupling the pricking needle device and the needle end protective cap together. The pricking needle device comprises position registration means, coupling means and a needle end housing section. The position registration means is provided on the pricking member body and on the needle end protective cap for effecting position registration therebetween, in such a manner that, when the pricking member body is coupled to the needle end protective cap, the pricking member body is guided to a preset location of the needle end protective cap. The coupling means includes an engagement segment and an engagement groove which are engaged with each other when the pricking member body and the needle end protective cap, the position registration of which has been effected by the position registration means, are rotated relative to each other. The needle end housing section is provided in the needle end protective cap and houses therein the distal end of the needle protruded from the end face of the pricking member body. The needle end housing section is closed with hermetic seal by the pricking member body when the distal end of the pricking member body is coupled to the needle end protective cap.
If, in coupling the pricking member body and the needle end protective cap together, the distal end of the pricking member body is inserted into the needle end protective cap, the pricking member body may be in register with the needle end protective cap by the operation of the position registration means. With the pricking member body in register with the needle end protective cap, the pricking member body and the needle end protective cap are rotated relative to each other until the engagement segment provided on the pricking member body is engaged with the engagement groove in the needle end protective cap, thereby coupling the pricking member body and the needle end protective cap to each other.
When the pricking member body and the needle end protective cap are coupled together in this manner, the distal end of the needle, protruded from the end face of the pricking member body, is housed in the needle end housing section provided in the needle end protective cap. The needle end housing section is hermetically sealed at this time by the pricking member body coupled to the needle end protective cap.
With the pricking needle device of the present invention, position registration between the pricking member body and the needle end protective cap may be realized by simply admitting the pricking member body into the needle end protective cap. The pricking member body and the needle end protective cap may then be coupled to each other by simply relatively rotating the pricking member body and the needle end protective cap. Hence, no larger force is needed to mount the needle end protective cap on the pricking member body.
Thus, the needle used for blood sampling may be accommodated with ease in the needle end protective cap, while no pain is inflicted on a person who has his/her blood sampled from finger end.
Moreover, when the needle end protective cap is mounted on the pricking member body, the distal end of the needle used for pricking the finger end is housed in a hermetically sealed state in the needle end housing section. Hence, the blood affixed to the needle may positively be prevented from leaking to outside to enable disposal in safety.
Other objects and advantages derived from the present invention will become more apparent from the following description which will now be made in conjunction with the accompanying drawings.

### Brief Description of the Drawings

Fig.1 is a perspective view of a conventional pricking needle device showing the state in which a needle end protective cap is being attached to a pricking member body.
Fig.2 is a perspective view showing an embodiment of a pricking needle device according to the present invention.
Fig.3 is a perspective view, looking from the bottom side, of the pricking needle device shown in Fig.2.
Fig.4 is a plan view showing the pricking needle device shown in Fig.2.
Fig.5 is a side view of the pricking needle device shown in Fig.2.
Fig.6 is a bottom view of the pricking needle device shown in Fig.2.
Fig.7 is a cross-sectional view taken along line VII-VII of Fig.4.
Fig.8 is a cross-sectional view taken along line VIII-VIII of Fig.6.
Fig.9 is a perspective view showing the distal end of the pricking member body as separated from the needle end protective cap.
Fig.10 is a perspective view showing the needle end protective cap.
Fig.11 is a cross-sectional view showing the state in which the pricking member body is being introduced into the needle end protective cap.
Fig.12 is a cross-sectional view showing the state in which the pricking member body has been introduced into the fitting recess of the needle end protective cap.
Fig.13 is a cross-sectional view showing the state in which an engagement segment on the pricking member body has been engaged in an engagement groove in the needle end protective cap.
Fig.14 is a cross-sectional view showing the state in which, after engaging the engagement segment of the pricking member body in the engagement groove in the needle end protective cap, the pricking member body and the needle end protective cap are coupled by relative rotation to each other.
Fig.15 is a perspective view showing the state in which the pricking member body is mounted on a lancet holder and the needle end protective cap is mounted in position.
Fig.16 is a perspective view showing the state in which the pricking member body has been caused to descend and thrust into the needle end protective cap.
Fig.17 is a perspective view showing the state in which the needle end protective cap is retained and picked up by the distal end of the pricking member body.
Fig.18 is a perspective view showing the state in which the pricking member body and the needle end protective cap have been gripped by fingers of both hands.
Fig.19 is a perspective view showing the state in which the pricking member body and the needle end protective cap are gripped and coupled together on relative rotation.
Fig.20 is a perspective view showing the state in which the pricking member body and the needle end protective cap have been coupled together on relative rotation.
Fig.21 is a perspective view, looking from the bottom side of the needle end protective cap, and showing the state in which the pricking member body and the needle end protective cap have been coupled together.
Fig.22 is a perspective view showing another embodiment of a pricking needle device according to the present invention.
Fig.23 is a perspective view of Fig.22, when looking from the bottom side of the pricking needle device.
Fig.24 is a perspective view showing another embodiment of a needle end protective cap.
Fig.25 is a cross-sectional view showing a case where the pricking member body and the needle end protective cap have been irreversibly coupled together.
Fig.26 is a perspective view showing a further embodiment of a needle end protective cap 13.

### Best Mode for Carrying out the Invention

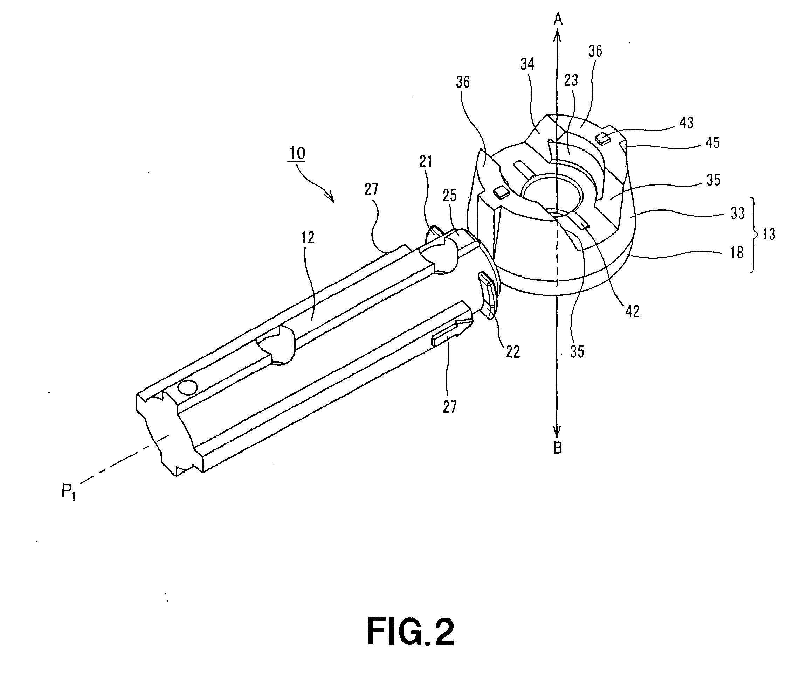

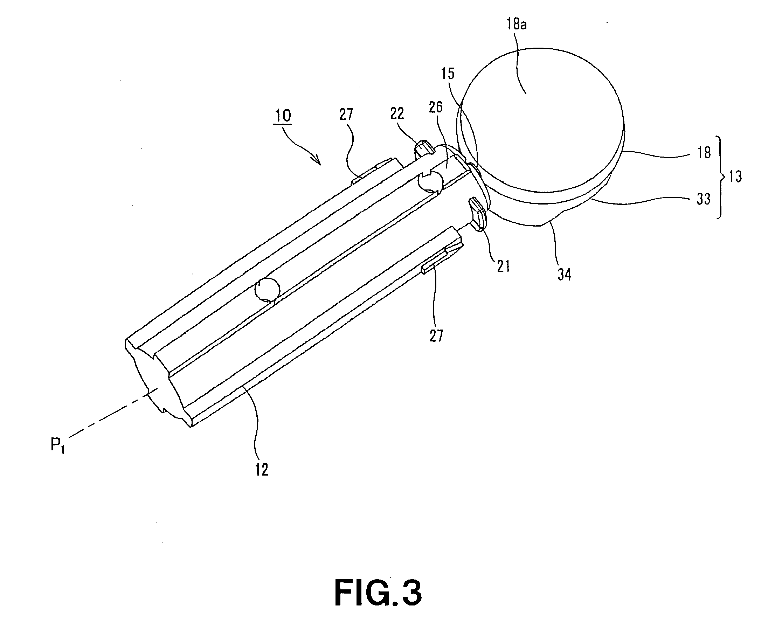

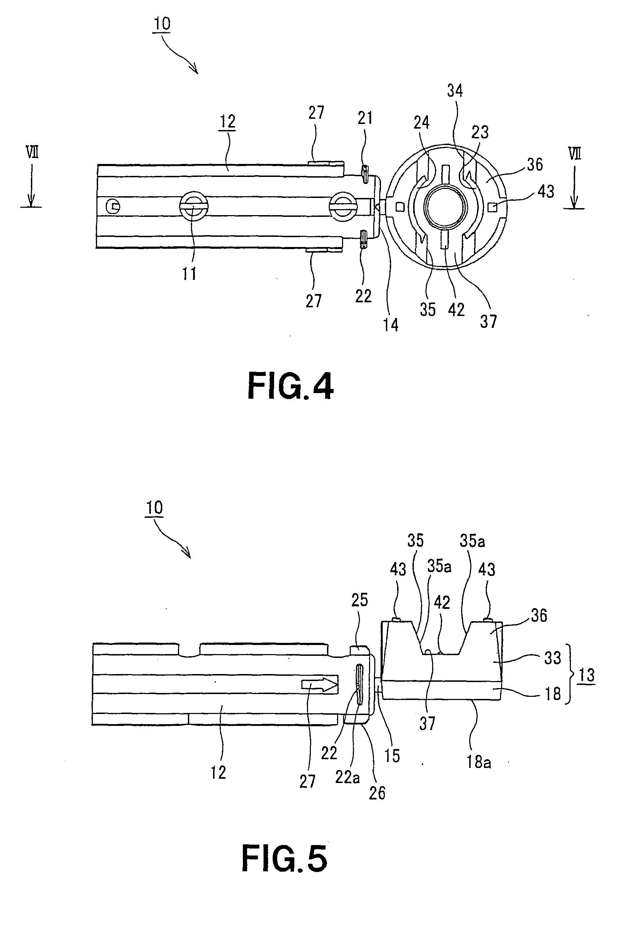

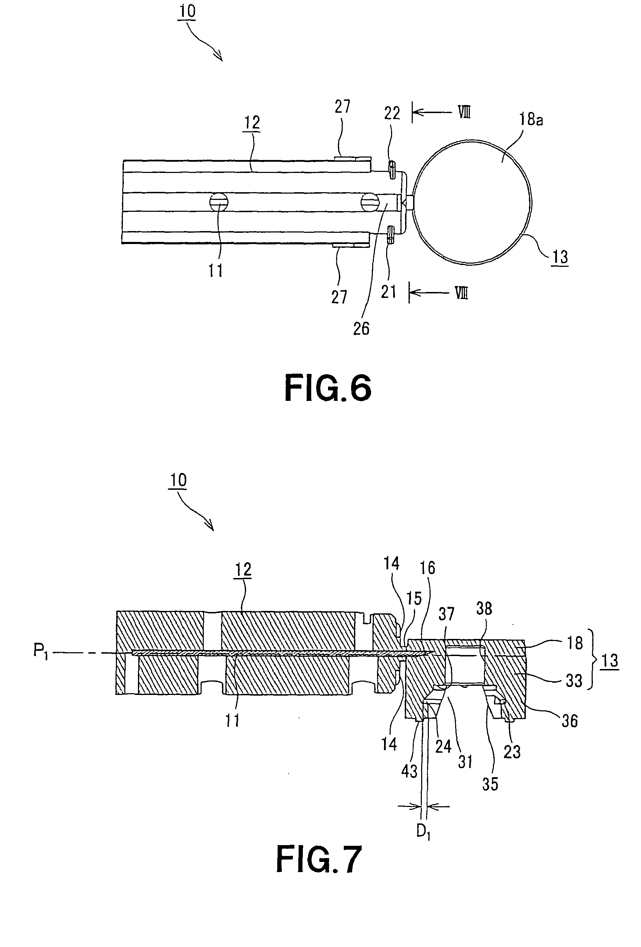

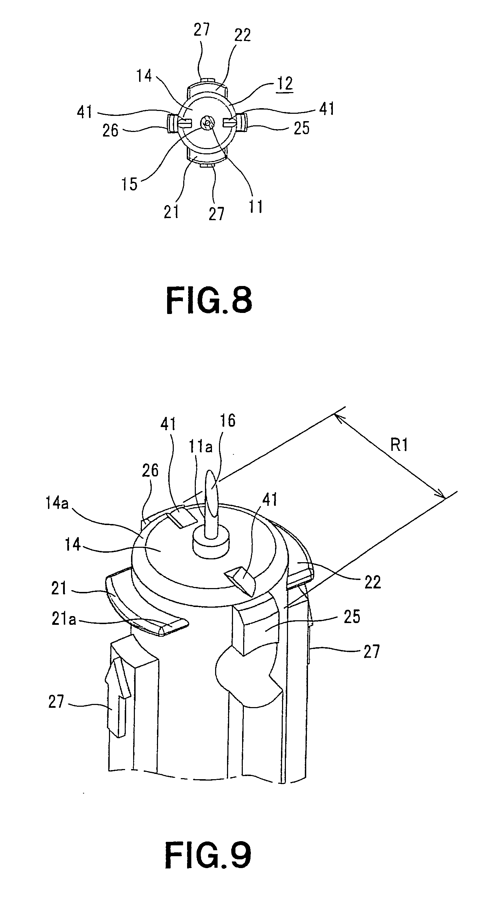

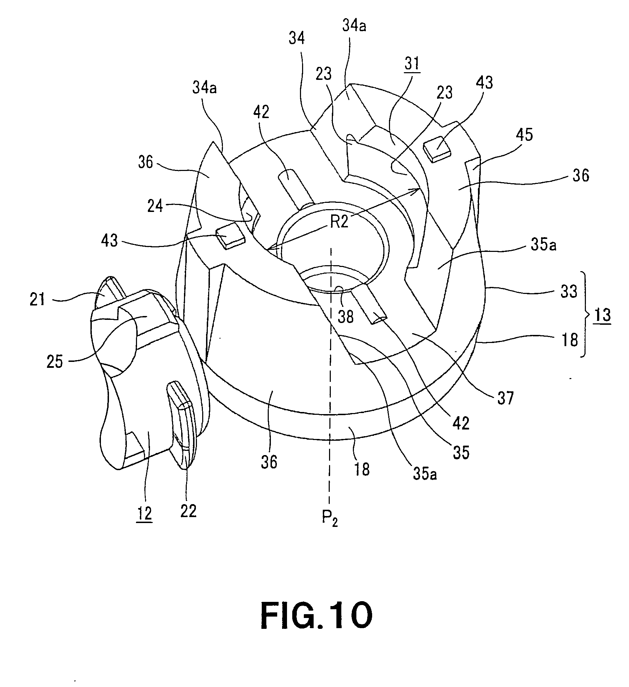

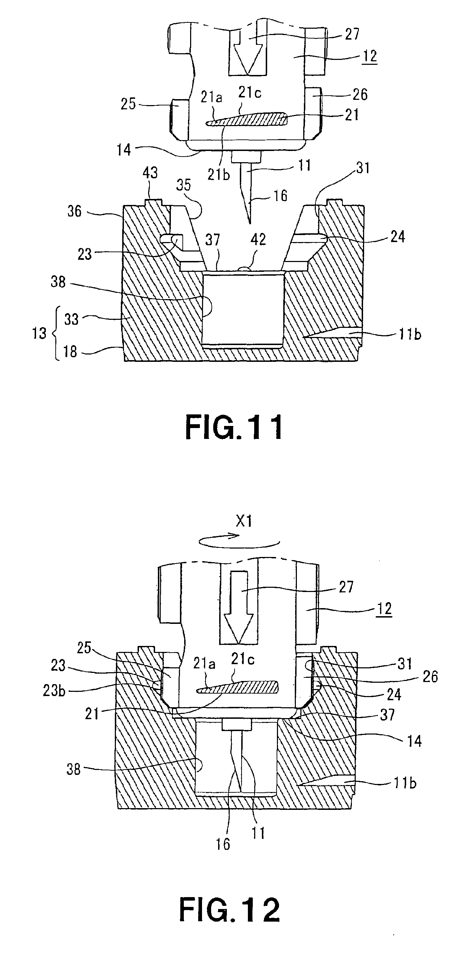

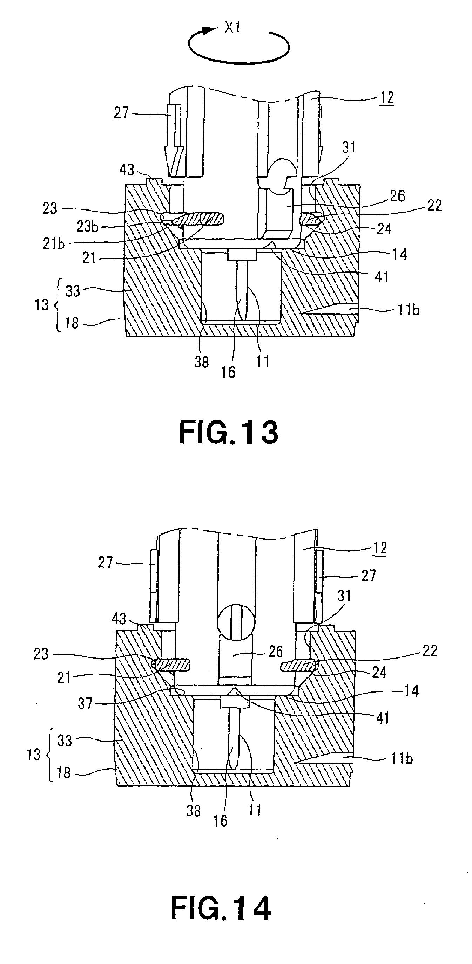

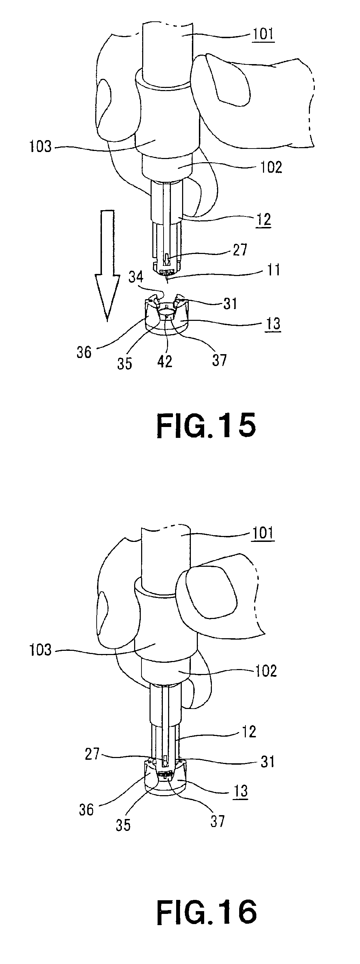

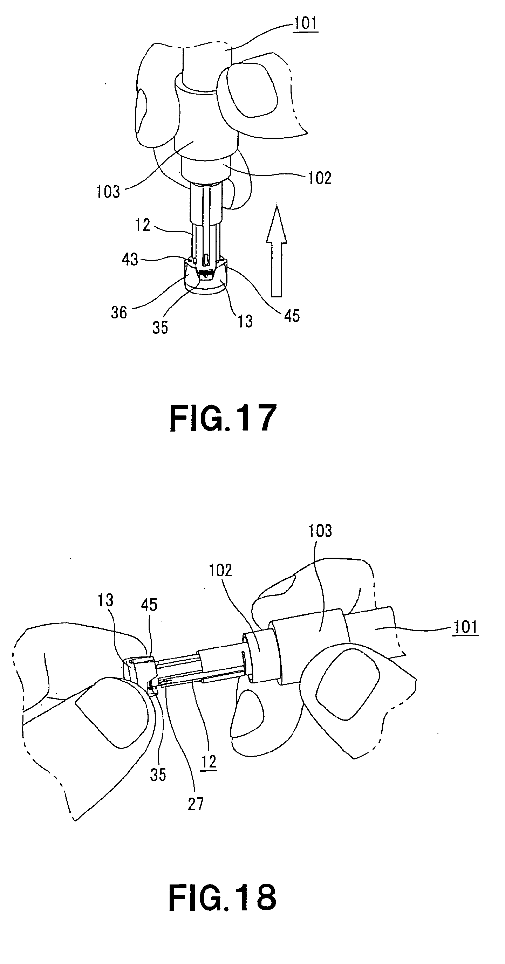

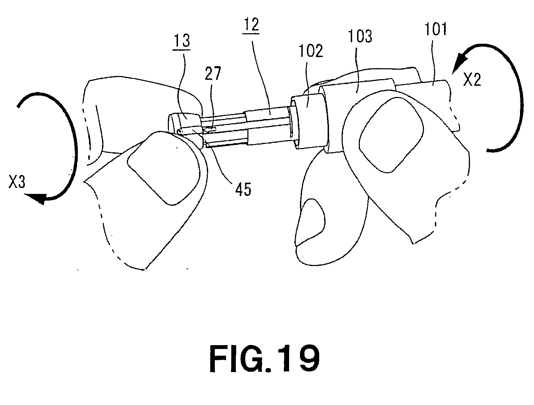

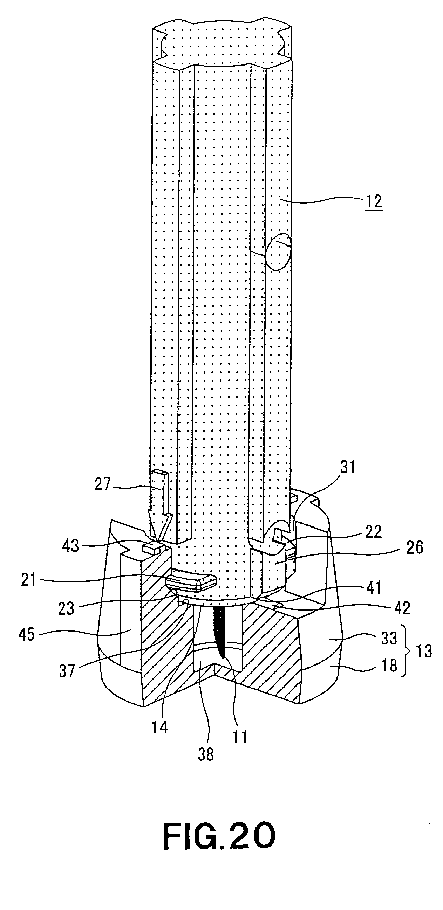

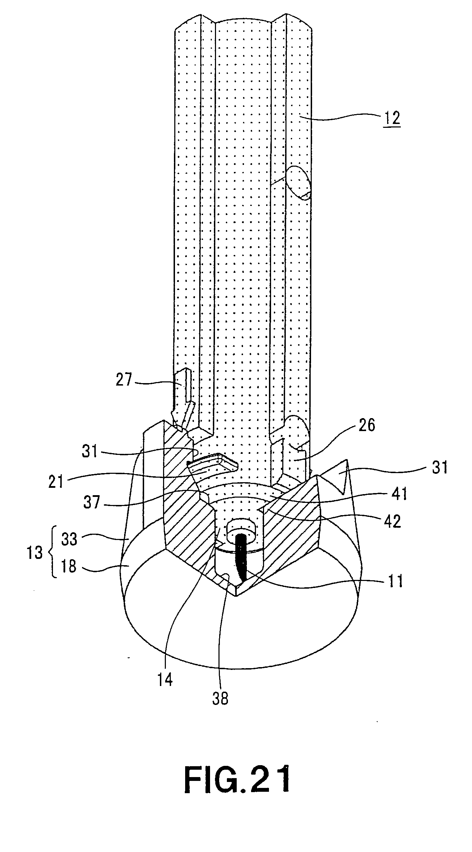

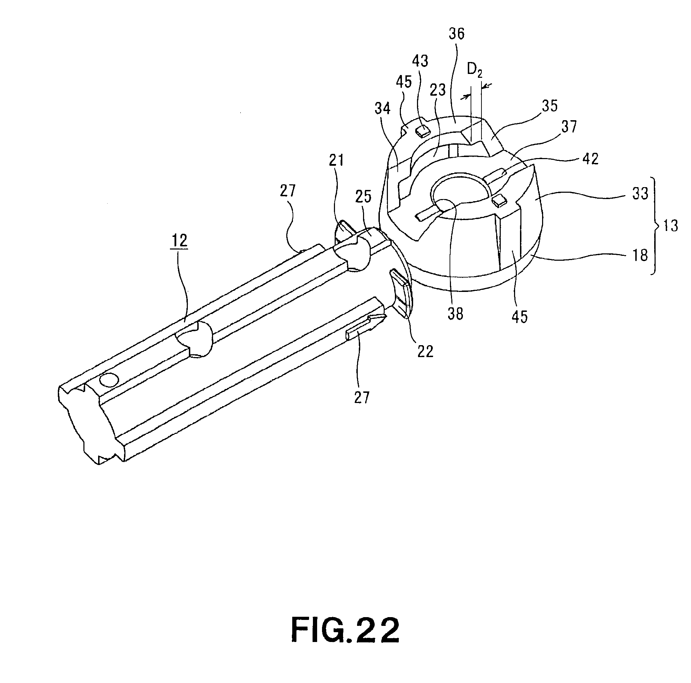

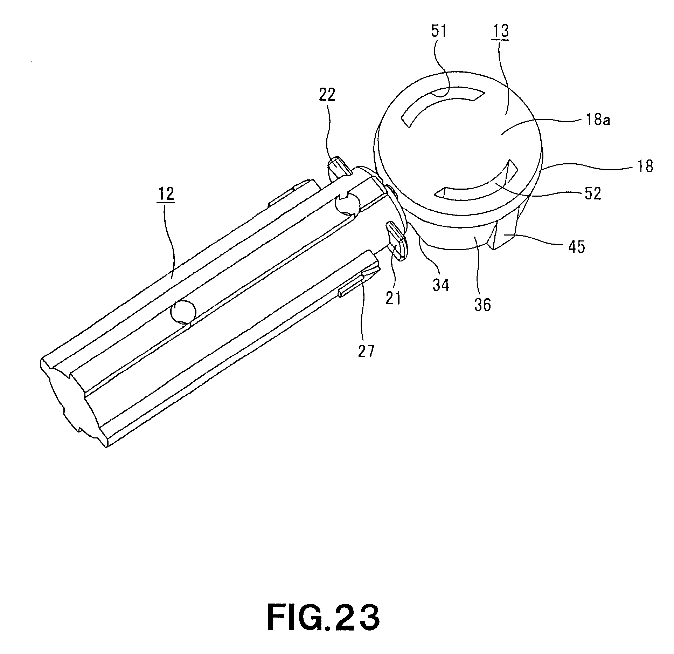

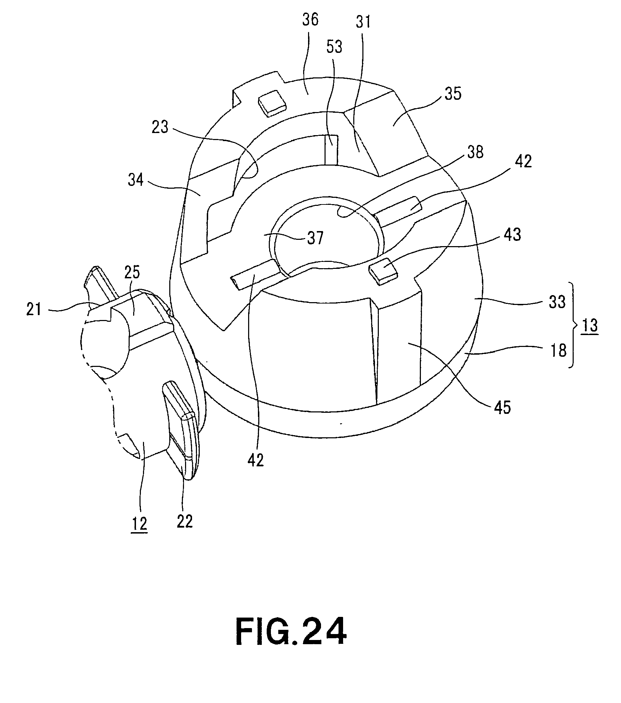

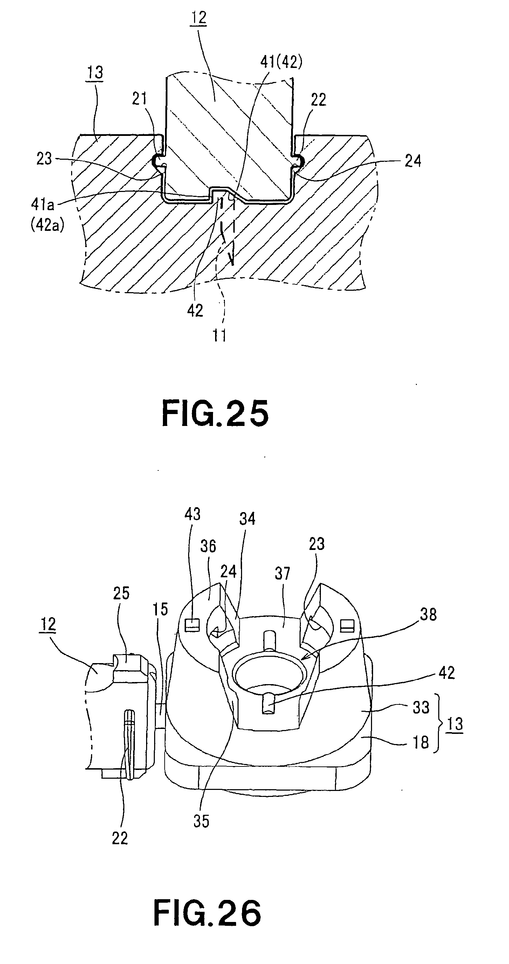

An embodiment of a pricking needle device according to the present invention will now be described with reference to the drawings.
In the present embodiment, the present invention is applied to a pricking needle device, termed a lancet, used by a diabetic to sample his/her blood to measure his/her own blood sugar value, for example. Referring to Figs.2 to 7, a pricking needle device 10 includes a needle 11, a pricking member body 12, integrally carrying the needle 11, and a needle end protective cap 13. The needle 11 is used to prick the finger end. The needle end protective cap is fitted to a distal end 11a of the needle 11 carried by the pricking member body 12.
The pricking member body 12 and the needle end protective cap 13 are unitarily molded from a synthetic resin material, using the same cavity of a metal mold of a molding device. For molding the pricking needle device 10, the needle is inserted into the cavity beforehand and a synthetic resin material is injected into the inside of the cavity.
The pricking member body 12 and the needle end protective cap 13 to be molded are unified together in such a state in which the distal end of the needle 11, protruded from an end face 14 of the pricking member body 12, pierces through a peripheral part of the needle end protective cap 13, as shown in Figs.2 to 7.
Meanwhile, in the present embodiment of the pricking needle device 10, the pricking member body 12 and the needle end protective cap 13 are not directly formed as one, but are maintained at a distance from each other, as shown in Figs.7 and 8. In actuality, the pricking member body and the needle end protective cap are unitarily connected to each other by a thin-walled connecting part 15 formed around the distal end 11a of the needle 11 which is protruded from the end face 14 of the pricking member body 12 to pierce through the peripheral part of the needle end protective cap 13. The pricking member body 12 and the needle end protective cap 13, thus interconnected, may be separated from each other by twisting the pricking member body 12 and the needle end protective cap 13 relative to each other, about the needle 11 as center, to sever the thin-walled connecting part 15. This thin-walled connecting part 15 is formed as a weakened portion for assisting in separation.
When the pricking member body 12 and the needle end protective cap 13 are separated from each other, the distal end 11a of the needle 11, piercing into the needle end protective cap 13, is extracted from the needle end protective cap 13 and protruded from the end face 14 of the pricking member body 12, as shown in Fig.9. That is, the needle 11 has been extracted from the needle end protective cap 13 as the needle is unitarily retained by the pricking member body 12.
The needle 11 has its major portion towards its proximal end embedded within the pricking member body 12, while it has its small length portion towards its distal end 11a piercing into the peripheral part of the needle end protective cap 13. Thus, when the pricking member body 12 and the needle end protective cap 13 are separated from each other, the needle is retained by the pricking member body 12. However, if it is desired to have the needle retained more reliably by the pricking member body 12, retention parts, not shown, such as protrusions or channels, may preferably be formed in the portions of the needle enclosed in the pricking member body 12 to prevent incidental extraction of the needle from within the pricking member body 12.
Before use of the pricking needle device 10, the pricking member body 12 and the needle end protective cap 13 are unified together. It is therefore possible to prevent the risk of loss of the needle end protective cap 13.
Moreover, the distal end 11a of the needle 11 for pricking the finger end of the human body, carrying a pricking part 16, is covered by the needle end protective cap 13, so that it is possible to prevent foreign matter from becoming deposited on the pricking part 16 before the needle pricks the finger end. Hence, the needle may prick in safety to permit the blood to flow in a minor quantity from the finger end.
The pricking needle device 10 of the present embodiment is now described in further detail.
The pricking needle device 10 of the present embodiment is used for pricking the finger end with the needle 11 to sample a minor quantity of blood, and hence is provided with a needle that may prick the skin without imparting severe pain to the user whose blood is being sampled. The needle 11 used herein is formed from a linear material 0.35 mm to 0.5 mm in diameter, and includes the acute pricking end 16, to permit easy pricking, as shown in Fig.9. This needle 11 is formed of a metal material, such as stainless steel.
The pricking member body 12, integrally holding the needle 11, is molded by injecting a synthetic resin material into a metal mold, as described above, and has the shape of a shank of a thickness and a length that may be gripped with three fingers, as shown in Figs.2 and 3. The needle 11 is carried by this pricking member body 12, as the needle has at least the distal side pricking part 16 protruded from the end face 14 of the pricking member body 12, and as the needle has its proximate side embedded in the pricking member body 12. The needle 11 is carried at this time as it is located along the center axis P 1 of the pricking member body 12, as shown in Fig.7. Also, the needle 11 is mounted with the distal end 11a carrying the pricking part 16 protruding about several mms from the end face 14 of the pricking member body 12. The portion of the needle protruded from the end face 14 of the pricking member body 12 pierces from the peripheral surface into a bottom part 18 of the needle end protective cap 13, as shown in Fig.7.
The rear side part of the pricking member body 12 opposite to its distal side, the pricking part 16 of the needle 11 is protruded from, has a shape and a size which will allow the rear side part to be mounted on a lancet holder of any of a large variety of commercially available projection instruments.
On the outer peripheral surface of the distal end of the pricking member body 12, the pricking part 16 of the needle 11 is protruded from, there are provided a pair of outwardly protruded engagement segments 21, 22, as shown in Figs.2 to 4. These engagement segments 21, 22 are formed at diametrically opposite positions about the center axis P1 of the pricking member body 12. When the needle end protective cap 13 is fitted on the distal end of the pricking member body 12, the engagement segments 21, 22 are engaged in engagement grooves 23, 24, provided in the needle end protective cap 13, respectively, to constitute a coupling mechanism for coupling the pricking member body 12 and the needle end protective cap 13 together.
Meanwhile, the engagement segments 21, 22 are wedge-shaped, with tapering distal ends 21a, 22a thereof intruded into the engagement grooves 23, 24, respectively, as shown in Fig.11.
The engagement segment 21, for example, out of the engagement segments 21, 22, has a side 21b towards the end face 14 extending parallel to this end face 14, while having the other side 21c inclined, thus presenting the wedge shape.
With the wedge-shaped engagement segments 21, 22, when the pricking member body 12 and the needle end protective cap 13 are rotated relative to each other so that the engagement segments 21, 22 are progressively engaged in the engagement grooves 23, 24, respectively, the engagement segments 21, 22 nip into the engagement grooves 23, 24, respectively, with an increasing engaging force, thus unifying the pricking member body 12 and the needle end protective cap 13 together.
At opposite positions on the outer peripheral surface towards the distal side of the pricking member body 12, there are formed a pair of rectangular protrusions 25, 26 extending along the axis of the pricking member body 12, as shown in Figs.2, 3 and 9. These protrusions 25, 26 are formed intermediate between the engagement segments 21, 22 protuberantly formed on the outer peripheral surface of the pricking member body 12. These protrusions 25, 26 constitute a thrusting supporting mechanism, operating so that, when the distal end side of the pricking member body 12 is introduced into a fitting opening 31 in the needle end protective cap 13, the protrusions are fitted in the fitting opening with a light thrusting force to enable the needle end protective cap 13 to be picked up by the pricking member body 12. That is, the diameter R1 of each of the zones of the pricking member body 12 provided with the protrusions 25, 26 is slightly larger than an inner diameter R2 of the fitting opening 31 in the needle end protective cap 13, by e.g. 0.02 to 0.05 mm.
On the outer peripheral surface towards the distal end of the pricking member body 12, there are formed a pair of indexes 27, 27 indicating the direction of admission when the pricking member body 12 is fitted into the inside of the needle end protective cap 13. These indexes 27, 27 are e.g. convex-shaped arrows integrally formed on the outer peripheral surface of the pricking member body 12.
The needle end protective cap 13, attached to the distal end of the pricking member body 12 in a state separated from the pricking member body 12, includes a substantially discoid bottom part 18, on one side of which is formed a fitting part 33, as shown in Figs.2 and 3. It is on this fitting part 33 that the pricking member body 12 is fitted and mounted in position. The fitting opening 31, in which the distal end of the pricking member body 12 has a fit, is formed centrally of the fitting part 33, as shown in Figs.2 and 10. It is by this fitting part 33 that the pricking member body 12 is fitted and mounted in position. The fitting opening 31 is formed as a circular recess having a center axis P2.
The fitting part 33 also includes a pair of engagement guides 34, 35 constituting a position registration mechanism for setting relative engagement positions between the pricking member body 12 and the needle end protective cap 13 when thrust fitting the distal end of the pricking member body 12 in the fitting opening 31. These engagement guides 34, 35, formed by partially removing a peripheral wall section 36 of the fitting opening 31, are engaged, at the time of thrust fitting, with the paired engagement segments 21, 22, respectively, to set relative engagement positions between the pricking member body 12 and the needle end protective cap 13 when the pricking member body 12 is fitted to the needle end protective cap 13.
Meanwhile, the engagement guides 34, 35 are formed with inclined facing surfaces 34a, 35a, respectively, these facing surfaces increasing in width towards the sides of the engagement guides into which are intruded the engagement segments 21, 22, such as to assure facilitated admission of the engagement segments 21, 22 into the engagement guides 34, 35. The engagement guides 34, 35 also operate for assuring correct setting of the admission position of the pricking member body 12 into the needle end protective cap 13.
In the inner peripheral surface of peripheral wall section 36, in which there is formed the fitting opening 31, there are formed the engagement grooves 23, 24 engaged by the engagement segments 21, 22, respectively. These engagement grooves 23, 24 are formed to an arcuate shape having the center axis P2 of the fitting opening 31 as center.
The engagement grooves 23, 24 are formed by a molding technique known as undercut molding.
The engagement grooves 23, 24 are formed so that, when the distal end side of the pricking member body 12 is admitted into the fitting opening 31, as the engagement segments 21, 22 are within the engagement grooves 23, 24, respectively, and the end face 14 is thrust against the bottom surface 37 of the fitting opening 31, opening ends 23a, 24a of the engagement grooves 23, 24 face one ends of the engagement segments 21, 22, respectively, and the side 23b of the groove 23 towards the bottom surface 37 is in registration with the side 21b towards the foremost part of the engagement segment 21, as shown in Figs.12 and 13.
With the above constitution of the engagement grooves 23, 24, the engagement segments 21, 22 may be spontaneously intruded into the engagement grooves 23, 24 by rotating the pricking member body 12, having its distal end admitted into the fitting opening 31, in a direction indicated by arrow X1 in Figs.12 and 13 relative to the needle end protective cap 13. At this time, the engagement segments 21, 22 are intruded into the engagement grooves 23, 24, respectively, as the pricking member body 12 is rotated, with the bottom surface 37 of the fitting opening 31, the end face 14 rests on, as a reference surface. The pricking member body 12 may be unified with the needle end protective cap 13, with the thickened portions towards the proximal sides of the engagement segments 21, 22 nipping into the engagement grooves 23, 24, as shown in Fig.14.
A circular needle end housing section 38 is formed centrally of the bottom surface 37 of the fitting opening 31. When the pricking member body 12 is coupled with the needle end protective cap 13, the needle end housing section 38 houses the distal end 11a of the needle 11, carrying the pricking part 16, protruded from the foremost part of the pricking member body 12, such as to isolate the distal end 11a from outside.
With the present embodiment of the pricking needle device 10, the distal end 11a of the needle 11, carrying the pricking part 16, may be housed in the needle end housing section 38 and may thus be isolated from outside, when the pricking member body 12 is coupled with the needle end protective cap 13. Hence, the blood, still affixed to the pricking part 16, may be prevented from being scattered to outside.
In the bottom surface 37 of the fitting opening 31, there are formed a pair of retention projections 42, 42 adapted to be engaged with a pair of retention recesses 41, 41, formed in the end face 14 of the pricking member body 12, when the pricking member body 12 is coupled with the needle end protective cap 13. These retention projections 42, 42 are formed at such positions that, when the pricking member body 12 and the needle end protective cap 13 are rotated relative to each other in a preset direction, subsequent to admission of the distal end side of the pricking member body 12 into the fitting opening 31 in the needle end protective cap 13, with the engagement segments 21, 22 then engaging in the engagement grooves 23, 24, the retention projections 42, 42 are engaged with the retention recesses 41, 41. With the retention projections 42, 42 thus engaged with the retention recesses 41, 41, the coupled state between the pricking member body 12 and the needle end protective cap 13 may be set. That is, the retention recesses 41, 41 in the pricking member body 12 and the retention projections 42, 42 on the needle end protective cap 13 constitute a coupling setting mechanism for prohibiting further relative rotation between the pricking member body 12 and the needle end protective cap 13 when the pricking member body and the needle end protective cap are once coupled together as described above.
Meanwhile, the position relationships between the retention recesses 41, 41 and the retention projections 42, 42 may be reversed from those described above, that is, the retention recesses 41, 41 may be provided in the needle end protective cap 13 and the retention projections 42, 42 may be provided on the pricking member body 12.
On the end face of the peripheral wall section 36 of the fitting opening 31, there are formed a pair of coupling position indicating marks 43, 43, indicating the relative coupling positions of the pricking member body 12 and the needle end protective cap 13, as shown in Fig.10. These coupling position indicating marks 43, 43 are provided at such positions that, when the pricking member body 12 and the needle end protective cap 13 are coupled together, with the engagement segments 21, 22 engaging with the engagement grooves 23, 24, the coupling position indicating marks 43, 43 are in register with the indexes 27, 27 provided on the pricking member body 12. That is, the coupling position indicating marks 43, 43 constitute coupling position indexing parts, for indicating that, when the pricking member body 12 and the needle end protective cap 13 are relatively rotated a preset amount, with the engagement segments 21, 22 engaging with the engagement grooves 23, 24, respectively, and the coupling position indicating marks 43, 43 are in register with the indexes 27, 27, the pricking member body 12 and the needle end protective cap 13 have been coupled together.
With the present embodiment of the pricking needle device 10, the coupling state between the pricking member body 12 and the needle end protective cap 13 may be confirmed not only by the feeling imparted to the finger end when the retention recesses 41, 41 and the retention projections 42, 42 are engaged with each other, but also with the position registration between the coupling position indicating marks 43, 43 and the indexes 27, 27. Hence, the coupled state between the pricking member body 12 and the needle end protective cap 13 may be confirmed straightforwardly.
It is noted that the bottom surface 18a of the bottom part 18 of the needle end protective cap 13 is formed as a planar surface, as shown for example in Figs.3 and 5, to permit the needle end protective cap 13, separated from the pricking member body 12, to be set in stability on a setting site, such as a table. It is sufficient that the bottom surface 18a of the bottom part 18 is substantially planar so that it may be set in stability on e.g. the table.
The sequence of operations for pricking the finger end, using the above-described pricking needle device 10 of the present embodiment, and the processing steps subsequent to pricking, are now described in detail.
To prick the finger end to sample the blood, using this pricking needle device 10, the pricking member body 12 and the needle end protective cap 13 are held with the finger end. The pricking member body 12 and the needle end protective cap 13 are twisted and relatively rotated about the needle 11 as center to sever the thin-walled connecting part 15 and separate the pricking member body 12 and the needle end protective cap 13 from each other.
At this time, the distal end 11a of the needle 11, having the pricking part 16, is protruded from the distal end of the pricking member body 12, as shown in Fig.9.
When the pricking member body 12 and the needle end protective cap 13 are separated from each other, there is left a mark 11b of extraction of the distal end 11a of the extracted needle 11 in the needle end protective cap 13, as shown in Figs.11 to 14.
The pricking member body 12, separated from the needle end protective cap 13, is attached to a well-known projection instrument 101, widely used as a blood sampling device.
The projection instrument 101 is commercially available and well-known, and hence is not explained herein in detail. Briefly, it includes a tubular lancet holder 102 into which is inserted and supported the tubular pricking member body 12, as shown in Fig.15. The pricking member body 12 is mounted on the projection instrument 101 as the rear end of the pricking member body opposite to its end, the needle 11 is protruded from, is admitted into the lancet holder 102.
The projection instrument 101 includes a projection mechanism for projecting the pricking member body 12, mounted on the lancet holder 102, when the finger end is to be pricked with the needle 11. The projection instrument 101 also includes an ejection lever, not shown, for ejecting the pricking member body 12, mounted on the lancet holder 102, from the lancet holder 102.
The pricking member body 12 is mounted on the projection instrument 101, as shown in Fig.15. The pricking part 16 on the distal end of the needle 11 is applied against the finger end. The projection mechanism, not shown, provided on this projection instrument 101, is then actuated for projecting the pricking member body 12 out of a housing 103 of the projection instrument 101 along with the lancet holder 102. The pricking part 16 pierces the finger end to cause outflow of a minor amount of the blood. The blood thus caused to flow out is sampled on a test paper sheet for measuring the blood sugar value.
To attach the needle end protective cap 13 to the pricking member body 12, the needle end protective cap 13 is set on a setting site, such as on a table, with the flat bottom surface 18 of the bottom part 18 as a setting surface, with the opening side of the fitting opening 31 upwards, as shown in Fig.15.
The pricking member body 12 is then positioned, with the distal end of the needle 11 facing the fitting opening 31 of the needle end protective cap 13, as shown in Fig. 15. The pricking member body 12 is gripped at this time as it is mounted on the lancet holder 102. In the present embodiment, the pricking member body 12 is loaded on the needle end protective cap 13 without dismounting the pricking member body 12 from the lancet holder 102.
The reason is that, with the pricking needle device 10 of the present embodiment, it is unnecessary to thrust the pricking member body 12 with large force against the needle end protective cap 13, in order to mount the pricking member body 12 on the needle end protective cap 13, as later described, so that no excessive force is applied to the projection instrument 101. If excessive force is applied to the projection instrument 101, there is stored the biasing force in the biasing member which is designed to project the lancet holder 102 from the housing 103. In such case, there is fear that the lancet holder 102, holding the pricking member body 12, is projected erroneously during the operation of mounting the needle end protective cap 13, thus possibly causing scattering of the blood still affixed to the needle 11. This risk may be averted with the present embodiment of the pricking needle device 10.
Then, with the needle 11 facing the fitting opening 31, the user holds the projection instrument 101 with his/her three fingers and, in this state, causes the distal end of the pricking member body 12 to be intruded into the fitting opening 31 in the needle end protective cap 13, as shown in Fig.16. The pricking member body 12 is intruded at this time into the fitting opening 31, with the engagement segments 21, 22 then engaging with the engagement guides 34, 35, respectively, as shown in Figs.16 and 11.
In the present embodiment, the end face 14 on the distal end of the pricking member body 12 is chamfered at 14a so as to be tapered towards the end face 14, as shown in Fig.9. Additionally, the fitting opening 31 is formed in the needle end protective cap so as to be enlarged in diameter towards the opening side. Thus, by roughly registering the engagement segments 21, 22 with the engagement guides 34, 35, respectively, the engagement segments 21, 22 may be in register with and engaged in the engagement guides 34, 35, respectively, with the pricking member body 12 then being fitted in the fitting opening 31.
With the distal end of the pricking member body 12 positioned in the fitting opening 31, the pricking member body is further intruded into the inside of the fitting opening 31 of the needle end protective cap 13, as the paired engagement segments 21, 22 are guided by the inclined facing surfaces 34a, 35a of the engagement guides 34, 35. The pricking member body 12 is admitted into the fitting opening 31 until the end face 14a of the pricking member body 12 is thrust against the retention projections 42, 42 on the bottom surface 37 of the fitting opening 31. At this time, the pricking member body 12 has been fitted as its center axis P1 coincides with the center axis P2 of the fitting opening 31. The distal end of the needle 11, carried with its center axis coincident with the center axis P1 of the pricking member body 12, is accommodated at this time within the needle end housing section 38, as shown in Figs.16 and 12.
When the distal end of the pricking member body 12 is admitted into the fitting opening 31, as the paired engagement segments 21, 22 are engaged with the engagement guides 34, 35, respectively, the paired protrusions 25, 26, the outer diameter R1 of which is set so as to be slightly larger than the inner diameter R2 of the fitting opening 31, are in light thrusting contact with the inner peripheral surface of the fitting opening 31. The result is that the needle end protective cap 13 is retained by the distal end of the pricking member body 12 and may be picked up as one with the pricking member body 12, as shown in Fig.17.
Meanwhile, with the present pricking needle device 10, the needle end protective cap 13 may be retained by the pricking member body 12 by a simple dropdown operation from the distal end side of the pricking member body 12 retained by the lancet holder 102 under the combined weight of the pricking member body and the lancet holder. This may be achieved by setting the force of resistance against light thrusting contact fitting of the paired protrusions 25, 26 on the inner peripheral surface of the fitting opening 31 so as to be slightly lesser than the combined weight of the pricking member body 12 and the lancet holder 102.
The condition under which the pricking member body 12 retained by the lancet holder 102 is thrust into and retained by the inside of the needle end protective cap 13 under the combined weight is now scrutinized in comparison with the case of the conventional projection instrument 101.
The weight of the conventional projection instrument 101 is 250 to 350g, whereas the force needed for thrusting the lancet holder 102 into the inside of the housing 103 against the bias of a biasing member, not shown, to render the lancet holder 102 ready for projection, is 700 to 750g. Thus, if the pricking member body 12, retained by the lancet holder 102, is to be thrust into the inside of and retained by the needle end protective cap 13, without the lancet holder 102 being readied for projection, the force applied to the lancet holder 102 is desirably at most 700g or less and, for safety, 500g or less. Since the weight of the projection instrument 101, inclusive of the lancet holder 102, is 250 to 350g, the thrusting force applied from the pricking member body 12 to the needle end protective cap 13 is desirably 150g or less. Thus, the outer diameter R1 of the paired protrusions 25, 26 and the inner diameter R2 of the fitting opening R2 are set to give this thrusting force.
It is noted that the weight of the needle end protective cap 13 of the present embodiment is of the order of 0.2g to 0.5g. Thus, the pressure of thrusting the pricking member body 12 into the needle end protective cap 13 is to be set to approximately 150g to 30g. By so doing, the needle end protective cap 13 may be picked up as one with the pricking member body 12, without the needle end protective cap 13 disengaging from the pricking member body 12, when the lancet holder 102 is gripped and the pricking member body 12 is hoisted vertically.
The pricking member body 12, retained by the lancet holder 102, is dropped down by the combined weight of the pricking member body 12 and the lancet holder 102 onto the needle end protective cap 13 as set on the setting site. Thus, the distal end of the pricking member body 12 is thrust into the fitting opening 31 until the distal end 14 of the pricking member body 12 is thrust against the bottom surface 37 of the fitting opening 31, as shown in Fig.12. The one ends of the engagement segments 21, 22 are then facing the opening ends 23a, 24a of the engagement grooves 23, 24, respectively.
The lancet holder 102, holding the pricking member body 12, is dropped down onto the needle end protective cap 13 to cause the needle end protective cap 13 to be thrust into the distal end of the pricking member body, as described above. The needle end protective cap 13 is then uplifted, along with the pricking member body 12. In this state, the needle end protective cap 13 is gripped with the hand or the finger different from that which has gripped the pricking member body 12, as shown in Fig.18. As the pricking member body 12 and the needle end protective cap 13 are gripped with fingers of both hands, the pricking member body 12 is rotated in a direction indicated by arrow X2 in Fig.9, while the needle end protective cap 13 is rotated in the direction of rotation opposite to that of the pricking member body 12, that is, in a direction indicated by arrow X3 in Fig.19. By so doing, the engagement segments 21, 22 are forced to be engaged in the engagement grooves 23, 24, respectively.
The operation of rotating the projection instrument 101, inclusive of the lancet holder 102, holding the pricking member body 12, relative to the needle end protective cap 13, for engaging the engagement segments 21, 22 in the engagement grooves 23, 24, respectively, may be facilitated as in the case of operating a screwdriver. In addition, the engagement segments 21, 22 may be engaged in the engagement grooves 23, 24, respectively, with a smaller force.
Meanwhile, an anti-slip protrusion 45 is provided on the outer peripheral surface of the needle end protective cap 13, to help prevent slip to permit positive holding with the user's finger.
After engaging the engagement segments 21, 22 in the engagement grooves 23, 24, respectively, the pricking member body 12 is rotated further in the direction indicated by arrow X2 in Fig.19. In this case, as the pricking member body 12 is rotated with the bottom surface 37 of the fitting opening 31, as a reference surface, the engagement segments 21, 22 are intruded into the engagement grooves 23, 24, respectively, as shown in Fig.13. The pricking member body 12 has its end face 14 thrust against the bottom surface 37. The thickened portions towards the proximal ends of the engagement segments 21, 22 are then caused to progressively nip into the engagement grooves 23, 24, respectively, as shown in Fig.14.
When the pricking member body 12 is rotated until the engagement segments 21, 22 are intruded by preset amounts into the engagement grooves 23, 24, respectively, the retention recesses 41, 41 in the end face 14 of the pricking member body 12 are engaged by the retention projections 42, 42, as shown in Figs.20 and 21. With the retention recesses 41, 41 engaged by the retention projections 42, 42, the positions of engagement of the engagement segments 21, 22 with the engagement grooves 23, 24 are set, with the distal end of the pricking member body 12 being linked to and holding the needle end protective cap 13.
When the distal end of the pricking member body 12 is linked to and holding the needle end protective cap 13, the distal end of the needle 11, protruding from the end face 14 of the pricking member body 12, is accommodated in the needle end housing section 38, as shown in Figs.20 and 21. At this time, the end face 14 of the pricking member body 12 is tightly contacted with the bottom surface 37 of the fitting opening 31. The needle end housing section 38 is closed in a hermetically sealed state. Hence, the distal end of the needle 11 is accommodated in the hermetically sealed needle end housing section 38, so that the needle 11 is prevented reliably from being exposed to outside. In addition, the body fluid, such as blood, affixed to the needle 11, may be reliably prevented from being scattered to outside.
In the present pricking needle device 10, the state in which the pricking member body 12 and the needle end protective cap 13 are coupled together, with the engagement segments 21, 22 fully engaging in the engagement grooves 23, 24, respectively, may be recognized by the tactile sensation at the finger end at the time of engagement between the retention recesses 41, 41 and the retention projections 42, 42. In addition, such state may be recognized visually by the indexes 27, 27 on the pricking member body 12 registering with the coupling position indicating marks 43, 43 on the needle end protective cap 13, as shown in Fig.20. With the present pricking needle device 10, the state in which the pricking member body 12 and the needle end protective cap 13 have been coupled together may be confirmed not only from the sensation on the user's fingers at the time of the rotating operation, but also visually. Hence, the operation of linking the pricking member body 12 and the needle end protective cap 13 together may be achieved without the necessity of carrying out excessive operations.
Moreover, with the present pricking needle device 10, as the pricking member body 12 is rotated relative to the needle end protective cap 13, and as the engagement segments 21, 22 are progressively engaged with the engagement grooves 23, 24, respectively, the center axis P1 of the pricking member body 12 becomes spontaneously confounded with the center axis P2 of the needle end protective cap 13, under the concerted action of the pricking member body 12 and the needle end protective cap 13, with the pricking member body 12 becoming progressively set upright with respect to the needle end protective cap 13.
Thus, with the present pricking needle device 10, a constant coupled state may be achieved at all times in the operation of coupling by relative rotation of the pricking member body 12 and the needle end protective cap 13, without difference in the skill on the part of users or without difference in the result of coupling or reliability in coupling.
In the present embodiment, it is targeted to relieve the load felt by the user's finger holding the needle end protective cap 13 during rotation of the pricking member body 12 relative to the needle end protective cap 13 for engaging the engagement segments 21, 22 in the engagement grooves 23, 24, respectively.
To this end, the pricking member body 12 and the needle end protective cap 13 are formed of polyethylene resin, which is crystalline resin exhibiting moderate elasticity and flexibility and having an extremely low dynamic frictional coefficient µ of 0.15 equivalent to that of the fluorine resin. Moreover, the angle of relative rotation between the pricking member body 12 and the needle end protective cap 13 for engaging the engagement segments 21, 22 in the engagement grooves 23, 24 for coupling the pricking member body 12 and the needle end protective cap 13 together is 90° or less, while the total distances the paired engagement grooves 23, 24 are rotated are as small as 9.4 mm (4.7mm × two places). In addition, the contact areas between the engagement segments 21, 22 and the engagement grooves 23, 24 are also small and equal to about 10 mm² (5 mm² × two places). Hence, no significant sensation of resistance may be imparted to the user's finger holding the needle end protective cap 13 during rotation of the pricking member body 12 relative to the needle end protective cap 13. That is, the pricking member body 12 and the needle end protective cap 13 may be coupled together by a light rotational operation.
It may occur that, during the process of coupling between the pricking member body 12 and the needle end protective cap 13, various parts of the pricking member body 12 and the needle end protective cap 13 may conflict with each other, or moderate deformation may be produced in riding over resistive portions. However, since the pricking member body 12 and the needle end protective cap 13 are formed of polyethtylene resin, which is a crystalline resin, the deformed state may be instantaneously vanished to restore to the original non-deformed state the instant the conflicting or resisting zone is leaped over. Hence, no defects are caused in the coupling mechanism or in the operation or effect of the present invention.
Moreover, since the pricking member body 12 and the needle end protective cap 13 are formed of a crystalline resin, for example, a polyethylene resin, which is restored to its original shape after becoming transiently deformed, the wedge-shaped engagement segments 21, 22 are nipped into the engagement grooves 23, 24, thereby realizing strong connection.
In the above embodiment, a depth D1 of the engagement grooves 23, 24, engaged by the engagement segments 21, 22 on the pricking member body 12, respectively, is shallow and is not larger than 0.2 mm, as shown in Fig.7. The reason is that a metal mold for molding the engagement grooves is released from the opening of the fitting opening 31, narrower than the distance between the engagement grooves 23, 24, by so-called forced mold release, thus imposing limitations on the depth of molding.
Thus, if it is necessary to deepen a depth D 1 of the engagement grooves 23, 24 and hence the depth of engagement thereof with the engagement segments 21, 22, the needle end protective cap 13 is molded using a pair of core pins for forming the engagement grooves. With the use of the core pins for forming the engagement grooves, it becomes unnecessary to force out the core pins, so that the depth D 1 of the engagement grooves 23, 24 shown in Fig.22 may be set freely. These core pins, used for forming the engagement grooves, are admitted from the bottom surface 18a of the bottom part 18 of the needle end protective cap 13 into contact with the inner peripheral surface of the peripheral wall section 36 constituting the fitting opening 31, as shown in Fig.23. A pair of through-holes 51, 52, through which the core pins for forming the engagement grooves 23, 24 are to be extracted, are formed in the bottom surface 18a of the bottom part 18 of the needle end protective cap 13, in which the engagement grooves 23, 24 have been formed using the core pins.
By deepening the depth D1 of the engagement grooves 23, 24 and increasing the size of the engagement segments 21, 22 to increase the depth of engagement therebetween, it is possible to realize stronger connection of the needle end protective cap 13 to the pricking member body 12.
If the needle end protective cap 13, inclusive of the engagement grooves 23, 24, is to be molded using the core pins for forming the engagement grooves, the engagement guides 34, 35 in the peripheral wall section 36 of the fitting opening 31 are formed so as to be arrayed along the axis of the pricking member body 12, as shown in Figs.22 and 23, in distinction from the case of the pricking needle device 10 described above with reference to Figs.2 and 3. This constitution is derived from the arraying of the core pins for forming the engagement grooves. That is, the connection relationships of the pricking member body 12 and the needle end protective cap 13 may suitably be selected depending on the constitution of the metal mold device designed for unitarily molding the pricking member body 12 and the needle end protective cap 13 together.
The ends of the engagement grooves 23, 24 opposite to those into which are protruded one ends of the engagement segments 21, 22 may be closed with end stops 52, as shown in Fig.24. With these end stops 53, it is possible to prohibit excess relative rotation between the pricking member body 12 and the needle end protective cap 13, in coupling the pricking member body 12 and the needle end protective cap 13 to each other, thus realizing positive coupling.
In this configuration, it is possible to prevent the pricking member body 12 and the needle end protective cap 13, once coupled together, from being again separated from each other by further relative rotation. Specifically, the shape of the retention recesses 41, 41 and the retention projections 42, 42 is selected so that the retention recesses and the retention projections are irreversibly engaged with one another, as shown in Fig.25. By so doing, the pricking member body 12 and the needle end protective cap 13, once coupled together, may reliably be prohibited from separation from each other. That is, by setting flanks 41a, 42a of the retention recesses 41, 41 and the retention projections 42, 42, opposite to the sides thereof progressively engaged together, as upstanding surfaces, it is possible to prohibit rotation of the engagement segments 21, 22 in a direction of disengaging from the engagement grooves 23, 24.
By providing the end stops 53 in the engagement grooves 23, 24 to prohibit further relative rotation between the pricking member body 12 and the needle end protective cap 13, unified together, in the same direction as the coupling direction, and by selecting the shapes of the retention recesses 41, 41 and the retention projections 42, 42 so as to provide for irreversible rotation, as described above, the pricking member body 12 and the needle end protective cap 13, once coupled together, may be prohibited more reliably from being separated from each other. Thus, the needle 11, used for pricking and then covered up by the needle end protective cap 13, may be prevented from being again exposed to outside. As a result, the body fluid, such as blood, affixed to the needle 11, may be prevented reliably from being scattered to outside.
The needle end protective cap 13, attached to the pricking member body 12, is set on the setting site, such as a table. Hence, to assure stable setting of the needle end protective cap 13 on the setting site, the bottom part 18 may be designed as a rectangular section larger in size than the fitting part 33, as shown in Fig.26.
Although a preferred embodiment of the present invention has so far been described, the present invention is not limited to the constitution described and may encompass needle pricking devices in which a pricking member body is dropped down into a fitting opening in the needle end protective cap, and in which the pricking member body and/or the needle end protective cap is/are rotated in a preset direction to interconnect the pricking member body and the needle end protective cap.
In the above-described method for interconnecting the pricking member body and the needle end protective cap after pricking the finger end, the pricking member body is mounted in position on a pricking needle holder of a projection instrument. However, the present invention is not dependent entirely on the projection instrument as to attachment and interconnection of the pricking member body and the needle end protective cap. Specifically, the pricking member body, ejected from the pricking needle holder of the projection instrument, may be held with the finger end, and the pricking member body may be set at a preset location of the needle end protective cap. The needle end protective cap may then be picked up and the two components may then be relatively rotated and engaged with each other.

## Claims

1. A pricking needle device comprising a pricking member body of synthetic resin, carrying a pricking needle, with the distal end of said pricking needle being projected from an end face of said pricking member body, and a needle end protective cap of synthetic resin, into which the foremost part of the pricking member body, the distal end of the needle is protruded from, is inserted for coupling the pricking needle device and the needle end protective cap together; said pricking needle device further comprising:
position registration means provided on said pricking member body and on said needle end protective cap for effecting position registration therebetween, so that, when the pricking member body is coupled to the needle end protective cap, said pricking member body is guided to a preset location of said needle end protective cap;
coupling means including an engagement segment and an engagement groove which are engaged with each other when said pricking member body and said needle end protective cap, the position registration of which has been effected by said position registration means, are rotated relative to each other; and
a needle end housing section provided in said needle end protective cap and housing therein the distal end of said needle protruded from the end face of said pricking member body; said needle end housing section being closed with hermetic seal by said pricking member body when the distal end of the pricking member body is coupled to said needle end protective cap.

2. The pricking needle device according to claim 1 wherein said position registration means includes an engagement segment protruded from an outer peripheral surface of the distal end side of said pricking member body and an engagement guide on said needle end protective cap, said engagement segment engaging with said engagement guide.

3. The pricking needle device according to claim 1 wherein said coupling means includes an engagement segment protruded from the outer peripheral surface towards the distal end of said pricking member body and an engagement groove in an inner peripheral surface of a fitting opening in said needle end protective cap; said engagement segment being engaged with said engagement groove.

4. The pricking needle device according to claim 3 wherein said engagement segment is formed for extending in the peripheral direction of said outer peripheral surface of said pricking member body and is shaped as a wedge with a tapering distal end in the direction of intrusion into said engagement groove; said engagement groove being formed for extending in the peripheral direction of an inner peripheral surface of said fitting opening; said engagement segment engaging with said engagement groove on relative rotation between said pricking member body and said needle end protective cap to interconnect said pricking member body and said needle end protective cap.

5. The pricking needle device according to claim 1 further comprising:
coupling state setting means including a retention recess and a retention projection engaged with each other to restrict the rotation between said pricking member body and said needle end protective cap to set the coupling state therebetween when said engagement segment and said engagement groove of said coupling means engage with each other to interconnect said pricking member body and said needle end protective cap.

6. The pricking needle device according to claim 5 wherein said coupling state setting means includes a retention recess and a retention projection provided between the end face of said pricking member body and a bottom surface of said fitting opening in said needle end protective cap in which is housed the distal end of said pricking member body.

7. The pricking needle device according to claim 5 wherein said retention recess and a retention projection of said coupling state setting means are shaped to render the rotation between said pricking member body and the needle end protective cap irreversible.

8. The pricking needle device according to claim 5 wherein said engagement groove in the inner peripheral surface of said fitting opening, engaged by said engagement segment projected from the outer peripheral surface towards the distal end of said pricking member body, has a part opposite to the intruding side of said engagement segment closed to restrict further relative rotation between said engagement segment and the engagement groove after engagement of said engagement segment with said engagement groove to a preset depth of engagement.

9. The pricking needle device according to claim 1 wherein the distal end of said pricking member body has a part larger in diameter than the inner diameter of said fitting opening to permit said part to be thrust with a preset thrusting contact force against the surface of said fitting opening in said needle end protective cap.

10. The pricking needle device according to claim 1 wherein a coupling position indicating mark for indicating the coupling position between said pricking member body and said needle end protective cap is provided between the outer peripheral surface of the distal end of said pricking member body and an upper end surface of said needle end protective cap into which the distal end of said pricking member body is admitted.

11. The pricking needle device according to claim 1 wherein said needle end protective cap has a substantially planar bottom surface to allow said needle end protective cap to be set on its own on a setting site.

12. The pricking needle device according to claim 1 wherein said pricking member body and the needle end protective cap are formed as one from synthetic resin, with a connecting part in-between, said connecting part being in the vicinity of the distal end of the needle protruded from the end face of said pricking member body, and wherein said connecting part is formed as a thin-walled synthetic resin part covering the outer peripheral part of said needle and operating as a weakened portion to assist in separation of said pricking member body and the needle end protective cap from each other.

13. The pricking needle device according to claim 1 wherein said pricking member body and said needle end protective cap are molded of crystalline synthetic resin or a synthetic resin obtained on copolymerization thereof.
